# EUROPÄISCHE PATENTANMELDUNG

(11) **EP 2 572 630 A1**
(43) Veröffentlichungstag der Anmeldung: **27.03.2013**
(21) Anmeldenummer: 11182268.0
(22) Anmeldetag: 22.09.2011
(51) Int. Cl.: A61B 5/00

(54) **Vorrichtung zur Erkennung einer Anomalie an einem Zahn**

(71) Anmelder: W & H Dentalwerk Bürmoos GmbH, 5111 Bürmoos (AT)
(72) Erfinder: Heinrich, Dr., Christoph, 5110 Oberndorf (AT)
(74) Vertreter: Benda, Ralf

(57) **Zusammenfassung**

Vorrichtung (1, 1', 1") zur Erkennung einer Anomalie an einem Zahn (4), insbesondere zur Erkennung von Karies, Plaque oder Zahnstein, und / oder zur Erkennung von gesundem Zahngewebe umfassend: Eine Beleuchtungsvorrichtung (2), die zur Abgabe von Strahlung (6) auf zumindest einen zu untersuchenden Zahn (4) ausgebildet ist, derart, dass der Zahn (4) Strahlung (5) abstrahlt, und ein Filter (3), das ausgebildet ist, von dem Zahn (4) abgestrahlte Strahlung (5) durchzulassen, deren Wellenlänge zum Nachweis einer Anomalie an einem Zahn (4) geeignet ist, wobei das Filter (3) ausgebildet ist, gleichzeitig zumindest zwei Wellenlängen oder Wellenlängenbänder (5A) der von dem Zahn (4) abgestrahlten Strahlung (5) durchzulassen und zumindest jeweils eine Wellenlänge oder ein Wellenlängenband (5B) zwischen den durchgelassenen Wellenlängen oder Wellenlängenbändern (5A) auszufiltern, so dass die Strahlung (5) nach Verlassen des Filters (3) zumindest zwei Wellenlängen oder Wellenlängenbänder (5A) aufweist, welche durch zumindest eine ausgefilterte Wellenlänge oder zumindest ein ausgefiltertes Wellenlängenband (5B) voneinander getrennt sind. Des Weiteren wird ein entsprechendes Verfahren zur Aufbereitung von Strahlung (5), welche von einem zu untersuchenden Zahn (4) abgestrahlt wird, beschrieben.

## Beschreibung

Die vorliegende Erfindung betrifft eine Vorrichtung zur Erkennung einer Anomalie an einem Zahn und / oder zur Erkennung von gesundem Zahngewebe gemäß dem Oberbegriff des Anspruchs 1.

Eine derartige Vorrichtung ist zum Beispiel aus der Patentschrift US 5,306,144 bekannt. Um das Erkennen der Anomalie, insbesondere von Karies, Plaque oder Zahnstein, zu erleichtern, wird vorgeschlagen, aus der von dem zu untersuchenden Zahn abgestrahlten Strahlung zwei Wellenlängenbereiche oder Wellenlängenbänder auszufiltern und deren Intensitäten zu vergleichen.

Das Ausfiltern der beiden Wellenlängenbereiche erfolgt gemäß einem ersten Ausführungsbeispiel mit einem drehbaren, durch einen Motor antreibbaren Filterrad, an dem zwei Filter vorgesehen sind, die unterschiedliche Wellenlängenbereich durchlassen. Wird das Filterrad in Drehung versetzt, dann wird abwechselnd einer der beiden Filter in den optischen Pfad der von dem zu untersuchenden Zahn abgestrahlten Strahlung bewegt. Eine anschließend an das Filterrad angeordnete Auswerteinheit erhält somit abwechselnd und zeitlich versetzt jeweils ein von dem jeweiligen Filter durchgelassenes Wellenlängenband.

In einem alternativen Ausführungsbeispiel wird vorgeschlagen, die von dem zu untersuchenden Zahn abgestrahlte Strahlung auf zwei Lichtleiter aufzuteilen, welche die Strahlung zwei getrennten (stationären) Filtern zuführen. Die jeweils von den beiden Filtern durchgelassenen Wellenlängenbereiche werden nach dem Durchtritt durch die beiden Filter über zwei getrennte optische Pfade zwei getrennten Auswerteeinheiten zugeleitet.

Die Nachteile der beiden im Vorstehenden beschriebenen Ausführungsbeispiele sind unter anderem der komplizierte Aufbau und damit verbunden der große Raumbedarf der Filtervorrichtungen einschließlich der Auswerteeinheiten, um zwei Wellenlängenbereiche oder Wellenlängenbänder zu erhalten und zu vergleichen. So wäre es zum Beispiel aufgrund des Motors und der drehbaren Filterscheibe oder aufgrund der zwei Lichtleiter schwierig, wenn nicht unmöglich, die Filtervorrichtungen in ein Brillengestell zu integrieren, wie dies von Anwendern oftmals gewünscht wird, so dass diese in unkomplizierter Weise direkt (ohne Zwischenschaltung elektrischer Detektoren, bildgebender Vorrichtungen etc.) Anomalien des zu untersuchenden Zahns erkennen oder Anomalien und gesundes Zahngewebe unterscheiden können.

Der vorliegenden Erfindung liegt daher die Aufgabe zugrunde, eine Vorrichtung zur Erkennung einer Anomalie an einem Zahn, insbesondere von Karies, Plaque oder Zahnstein, und / oder zur Erkennung von gesundem Zahngewebe zu schaffen, welche aus der von dem zu untersuchenden Zahn abgestrahlten Strahlung zwei Wellenlängen oder Wellenlängenbänder ausfiltern kann, ohne die im Vorstehenden genannten Nachteile aufzuweisen. Die Vorrichtung soll insbesondere möglichst klein, von geringem Gewicht, einfachem Aufbau und von Anwender unkompliziert handhabbar sein.

Diese Aufgabe wird gemäß einem Ausführungsbeispiel durch eine Vorrichtung zur Erkennung einer Anomalie an einem Zahn, insbesondere von Karies, Plaque oder Zahnstein, und / oder zur Erkennung von gesundem Zahngewebe gelöst, welche aufweist: Eine Beleuchtungsvorrichtung, die zur Abgabe von Strahlung auf zumindest einen zu untersuchenden Zahn ausgebildet ist, derart, dass der bestrahlte, zu untersuchende Zahn Strahlung abstrahlt, und ein Filter, das ausgebildet ist, von dem zu untersuchenden Zahn abgestrahlte Strahlung durchzulassen, deren Wellenlänge zum Nachweis einer Anomalie an einem Zahn geeignet ist, wobei das Filter ausgebildet ist, gleichzeitig zumindest zwei Wellenlängen oder Wellenlängenbänder der von dem zu untersuchenden Zahn abgestrahlten Strahlung durchzulassen und zumindest jeweils eine Wellenlänge oder ein Wellenlängenband auszufiltern (oder zu unterdrücken oder abzuschwächen), welche(s) sich zwischen den durchgelassenen Wellenlängen oder Wellenlängenbändern befindet, so dass die Strahlung nach Verlassen des Filters zumindest zwei Wellenlängen oder Wellenlängenbänder aufweist, welche durch zumindest eine ausgefilterte Wellenlänge oder zumindest ein ausgefiltertes Wellenlängenband voneinander getrennt sind.

Das Vorsehen des im Vorstehenden beschriebenen Filters, das gleichzeitig zumindest zwei voneinander getrennte Wellenlängen oder Wellenlängenbänder durchlässt, verbessert, vereinfacht und verkleinert in vorteilhafter Weise erheblich den Aufbau der gesamten Vorrichtung zur Erkennung einer Anomalie an einem Zahn und / oder zur Erkennung von gesundem Zahngewebe. Insbesondere wird so dem Anwender ermöglicht, direkt (ohne Zwischenschaltung elektrischer Detektoren, bildgebender Vorrichtungen etc.) den zu untersuchenden Zahn zu beobachten und aufgrund der zumindest zwei voneinander getrennte Wellenlängen oder Wellenlängenbänder in merklich verbesserter Qualität, insbesondere bei erhöhtem Kontrast, Anomalien an dem Zahn zu erkennen und Anomalien und gesundes Zahngewebe zu unterscheiden.

Vorzugsweise ist die Transmission des Filters für die ausgefilterte Wellenlänge oder das ausgefilterte Wellenlängenband geringer als für die zumindest zwei durchgelassenen Wellenlängen oder für die zumindest zwei durchgelassenen Wellenlängenbänder und / oder das Filter ist derart ausgebildet, dass die Strahlungsintensität der ausgefilterten Wellenlänge oder des ausgefilterten Wellenlängenbands nach dem Filter geringer ist als die Strahlungsintensitäten der zumindest zwei durchgelassenen Wellenlängen oder der zumindest zwei durchgelassenen Wellenlängenbänder. Dies ist insbesondere unabhängig davon, ob die Transmission des Filters für die zumindest zwei durchgelassenen Wellenlängen oder für die zumindest zwei durchgelassenen Wellenlängenbänder in etwa gleich oder unterschiedlich ist, und unabhängig davon, ob die Strahlungsintensitäten der zumindest zwei durchgelassenen Wellenlängen oder der zumindest zwei durchgelassenen Wellenlängenbänder nach Verlassen des Filters in etwa gleich oder unterschiedlich sind,

Vorzugsweise ist das Filter ausgebildet, zumindest jeweils eine Wellenlänge oder ein Wellenlängenband, welche(s) sich zwischen den durchgelassenen Wellenlängen oder Wellenlängenbändern befindet, im Wesentlichen vollständig auszufiltern (oder zu unterdrücken oder abzuschwächen), so dass die Strahlung nach Verlassen des Filters zumindest zwei Wellenlängen oder Wellenlängenbänder aufweist, welche durch zumindest eine im Wesentlichen vollständig ausgefilterte Wellenlänge oder zumindest ein im Wesentlichen vollständig ausgefiltertes Wellenlängenband voneinander getrennt sind. Die Strahlungsintensität der im Wesentlichen vollständig ausgefilterten Wellenlänge oder des im Wesentlichen vollständig ausgefilterten Wellenlängenbands ist nach dem Durchtritt der vom Zahn abgestrahlten Strahlung durch den Filter auf vorzugsweise unter 5%, insbesondere unter 3%, der Strahlungsintensität vor dem Filter reduziert. Alternativ oder zusätzlich beträgt die Transmission des Filters für die zumindest eine Wellenlänge oder das eine Wellenlängenband, welche(s) sich zwischen den durchgelassenen Wellenlängen oder Wellenlängenbändern befindet und im Wesentlichen vollständig ausgefiltert wird, weniger als 5%, insbesondere weniger als 3%. Eine derartige, im Wesentlichen vollständige Ausfilterung wird zum Beispiel durch ein Interferenzfilter erzielt. Die Transmission eines derartigen Interferenzfilters beträgt für die zumindest zwei durchgelassenen Wellenlängen oder die zumindest zwei durchgelassenen Wellenlängenbänder vorzugsweise mehr als etwa 90%, insbesondere mehr als etwa 95%.

Alternativ ist das Filter ausgebildet, zumindest jeweils eine Wellenlänge oder ein Wellenlängenband, welche(s) sich zwischen den durchgelassenen Wellenlängen oder Wellenlängenbändern befindet, derart auszufiltern, dass die Strahlung nach Verlassen des Filters zumindest zwei Wellenlängen oder Wellenlängenbänder aufweist, welche durch zumindest eine ausgefilterte Wellenlänge oder zumindest ein ausgefiltertes Wellenlängenband voneinander getrennt sind, deren / dessen Strahlungsintensität nach dem Filter, insbesondere merklich oder signifikant, verringert wurde. Insbesondere ist die Strahlungsintensität der ausgefilterten Wellenlänge oder des ausgefilterten Wellenlängenbands, insbesondere merklich oder signifikant, geringer als die Strahlungsintensität der durchgelassenen Wellenlängen oder der durchgelassenen Wellenlängenbänder, zwischen denen sich die ausgefilterte Wellenlänge oder des ausgefilterte Wellenlängenband befindet. Die Strahlungsintensität der ausgefilterten Wellenlänge oder des ausgefilterten Wellenlängenbands ist nach dem Durchtritt der vom Zahn abgestrahlten Strahlung durch den Filter zum Beispiel um mehr als 50%, bevorzugt um mehr als 70%, besonders bevorzugt um mehr als 90%, geringer als die Strahlungsintensität zumindest einer der beiden durchgelassenen Wellenlängen oder Wellenlängenbänder, zwischen denen sich die ausgefilterte Wellenlänge oder des ausgefilterte Wellenlängenband befindet. Alternativ oder zusätzlich beträgt die Transmission des Filters für die zumindest eine ausgefilterte Wellenlänge oder das eine Wellenlängenband, welche(s) sich zwischen den durchgelassenen Wellenlängen oder Wellenlängenbändern befindet, weniger als 50%, bevorzugt weniger als 70%, besonders bevorzugt weniger als 90%.

Vorzugsweise umfasst die Beleuchtungsvorrichtung eine Strahlungsquelle, bevorzugt ein Halbleiterelement, zum Beispiel eine Leuchtdiode, insbesondere einer Laserdiode. Bevorzugt emittiert die Beleuchtungsvorrichtung elektromagnetische Strahlung, welche eine oder mehrere Wellenlängen im Bereich von etwa 380 nm - 420 nm umfasst. Vorzugsweise weist die Beleuchtungsvorrichtung neben der Strahlungsquelle, insbesondere dem Halbleiterelement, zumindest ein weiteres optisches Element auf, zum Beispiel einen Strahlungs- oder Lichtleiter, insbesondere einen Glasstab oder einen Glasfaserstab, einen Reflektor, welcher zumindest einen Teil der von der Strahlungsquelle abgestrahlten Strahlung in Richtung des zu untersuchenden Zahns reflektiert, oder eine optische Linse.

Vorzugsweise bildet das Filter einen, insbesondere einzigen oder gemeinsamen, optischen Pfad für die zumindest zwei durchgelassenen, voneinander getrennten Wellenlängen oder Wellenlängenbänder. Alternativ oder zusätzlich ist ein einziger, unmittelbar an das Filter anschließender, gemeinsamer optischer Pfad für die zumindest zwei durchgelassenen, voneinander getrennten Wellenlängen oder Wellenlängenbänder vorgesehen. Durch beide Maßnahmen wird der Aufbau der Vorrichtung zur Erkennung einer Anomalie an einem Zahn noch kleiner und einfacher.

Vorzugsweise ist das Filter als Mehrfach-Band-Pass-Filter ausgebildet, d.h. als Filter, welches mehrere voneinander getrennte Wellenlängen oder Wellenlängenbänder durchlässt, insbesondere als Doppel-Band-Pass-Filter zum Durchlassen von zwei voneinander getrennten Wellenlängen oder Wellenlängenbändern.

Beispielhaft umfassen die zumindest zwei von dem Filter durchgelassenen Wellenlängenbänder in etwa zwei der folgenden Bereiche: 480 nm - 490 nm; 480 nm - 550 nm; 510 nm - 530 nm; 540 nm - 550 nm; 600 nm - 670 nm.

Um Anomalien an einem Zahn besonders deutlich zu erkennen oder Anomalien und gesundes Zahngewebe besonders deutlich zu unterscheiden, ist gemäß bevorzugten Ausführungsbeispielen einer der folgenden Filter geeignet:
1. Ein Doppel-Band-Pass-Filter, das ein grünes und ein davon getrenntes rotes Wellenlängenband durchlässt, zum Beispiel Wellenlängen von etwa 510 nm - 530 nm und 600 nm - 670 nm;
2. Ein Doppel-Band-Pass-Filter, das ein blau-grünes und ein davon getrenntes rotes Wellenlängenband durchlässt, zum Beispiel Wellenlängen von etwa 480 nm - 550 nm und 600 nm - 670 nm;
3. Ein Mehrfach-Band-Pass-Filter (Tripel-Band-Pass-Filter), das ein blaues, grünes und eine rotes Wellenlängenband durchlässt, die jeweils voneinander getrennt sind, zum Beispiel Wellenlängen von etwa 480 nm - 490 nm, 540 nm - 550 nm und 600 nm - 670 nm.

Selbstverständlich sind die im Vorstehenden genannten Wellenlängenbänder und die Kombinationen der Wellenlängenbänder rein beispielhaft und es sind klarerweise auch andere Wellenlängenbänder oder andere Grenzen von Wellenlängenbändern oder andere Kombinationen von Wellenlängenbändern möglich.

Die Transmission des Filters sollte für die durchgelassenen Wellenlängen oder Wellenlängenbänder idealerweise möglichst groß sein, vorzugsweise liegt die Transmission für zumindest eine durchgelassene Wellenlänge oder ein durchgelassenes Wellenlängenband im Bereich von größer etwa 60% der auf das Filter auftreffenden Strahlung, besonders bevorzugt, insbesondere bei schmalen Wellenlängenbändern, im Bereich von größer etwa 75% oder 90%. Gemäß einem Ausführungsbeispiel ist die Transmission des Filters für die zumindest zwei durchgelassenen Wellenlängen oder die zumindest zwei durchgelassenen Wellenlängenbänder unterschiedlich, insbesondere ist die Transmission des Filters für die kurzwellige, durchgelassene Wellenlänge oder das kurzwellige, durchgelassene Wellenlängenband geringer als für die langwellige, durchgelassene Wellenlänge oder das langwellige, durchgelassene Wellenlängenband. Beispielsweise beträgt die Transmission des Filters für die kurzwellige, durchgelassene Wellenlänge oder das kurzwellige, durchgelassene Wellenlängenband unter etwa 50% und die Transmission des Filters für die langwellige, durchgelassene Wellenlänge oder das langwellige, durchgelassene Wellenlängenband mehr als etwa 70%. Selbstverständlich kann die Transmission des Filters für die beiden durchgelassenen Wellenlängen oder durchgelassenen Wellenlängenbänder auch in etwa gleich sein.

Ähnliches trifft auf die Strahlungsintensität der durchgelassenen Wellenlängen oder durchgelassenen Wellenlängenbänder zu: Gemäß einem Ausführungsbeispiel sind die Strahlungsintensitäten der zumindest zwei durchgelassenen Wellenlängen oder der zumindest zwei durchgelassenen Wellenlängenbänder nach Verlassen des Filters unterschiedlich, insbesondere ist die Strahlungsintensität der kurzwelligen, durchgelassenen Wellenlänge oder des kurzwelligen, durchgelassenen Wellenlängenbands geringer als die Strahlungsintensität der langwelligen, durchgelassenen Wellenlänge oder des langwelligen, durchgelassenen Wellenlängenbands. Selbstverständlich können die Strahlungsintensitäten der zumindest zwei durchgelassenen Wellenlängen oder der zumindest zwei durchgelassenen Wellenlängenbänder nach dem Filter jedoch auch in etwa gleich sein.

Vorzugsweise weist das Filter zumindest ein, vorzugsweise folienartiges, Trägermaterial auf, zum Beispiel Glas, Kunststoff oder Gelatine, auf welchem zumindest eine dielektrische Schicht aufgetragen ist oder welches mit Farbpigmenten versehen ist. Das Filter ist zum Beispiel aus farbigem Glas oder aus farbigem Kunststoff hergestellt. Alternativ oder zusätzlich kann das Filter zum Beispiel zumindest eine dielektrische Schicht aufweisen, es kann insbesondere als Filter mit einem Trägermaterial aus Kunststoff oder Glas ausgebildet sein, auf dem eine dielektrische Schicht aufgetragen ist. Vorzugsweise, insbesondere zur weiteren Reduktion des Gewichts und des Platzbedarfs, ist das Filter als farbige Folie ausgebildet, zum Beispiel als Kunststofffolie oder als gelatinehältige Folie mit Farbpigmenten. Klarerweise kann das Filter auch anders aufgebaut sein oder aus Kombinationen der im Vorstehenden genannten Materialien bestehen, zum Beispiel aus einem farbigem Glas und einer farbigen Filterfolie.

Vorzugsweise ist an dem Filter eine Vorrichtung oder ein Mittel zum Befestigen des Filters an einem Anwender der Vorrichtung zur Erkennung einer Anomalie an einem Zahn und / oder zur Erkennung von gesundem Zahngewebe oder eine Vorrichtung oder ein Mittel zum Halten durch den Anwender vorgesehen, zum Beispiel ein Brillengestell oder ein Griffteil. Damit kann der Anwender in vorteilhafter Weise den zu untersuchenden Zahn direkt (ohne Zwischenschaltung elektrischer Detektoren, bildgebender Vorrichtungen etc.) beobachten, wobei insbesondere durch die Vorrichtung zum Befestigen des Filters an einem Anwender der Anwender das Filter nicht mit der Hand halten muss und somit beide Hände für die andere Tätigkeiten frei hat. Bevorzugt weisen das Filter oder die Haltevorrichtung oder die Befestigungsvorrichtung einen Rahmen auf, der das Filter zumindest teilweise umgibt oder hält oder der insbesondere die Haltevorrichtung oder die Befestigungsvorrichtung mit dem Filter verbindet.

Vorzugsweise ist das Filter beweglich ausgebildet, insbesondere beweglich relativ zu zumindest einem Teil der Befestigungsvorrichtung zum Befestigen des Filters an dem Anwender oder der Vorrichtung zum Halten durch den Anwender, so dass das Filter in den optischen Pfad der von dem zu untersuchenden Zahn abgestrahlten Strahlung bewegbar und daraus entfernbar ist und / oder das Filter vor das Auge des Anwenders und vom Auge weg bewegbar ist. Damit kann der Anwender in vorteilhafter Weise während einer Behandlung wahlweise das Filter in den optischen Pfad und / oder vor sein Auge bewegen, um Anomalien zu erkennen oder Anomalien und gesundes Zahngewebe zu unterscheiden, oder aus dem optischen Pfad und / oder von seinem Auge weg bewegen, sobald er die Filterfunktion des Filters nicht benötigt. Bevorzugt ist das Filter beweglich, zum Beispiel klappbar, auf einem am Anwender befestigbaren Träger oder Gestell, zum Beispiel auf einem Brillengestell, befestigt, so dass der Anwender das Filter vor das Auge klappen kann oder es vom Auge entfernen kann.

Vorzugsweise ist / sind das Filter und / oder die Vorrichtung zum Befestigen des Filters an dem Anwender und / oder die Vorrichtung zum Halten durch den Anwender und / oder ein Betätigungselement zum Bewegen des Filters als Einwegvorrichtung(en) ausgebildet. Damit wird in vorteilhafter Weise eine Kreuzkontamination zwischen Patienten vermieden, wenn der Anwender während der Behandlung mit zum Beispiel einem unsterilen Handschuh das Filter, die Befestigungsvorrichtung, die Haltevorrichtung oder das Betätigungselement berührt.

Vorzugsweise weist die Vorrichtung zur Erkennung einer Anomalie am Zahn und / oder zur Erkennung von gesundem Zahngewebe ein Handgriffelement auf, in welchem zumindest ein Teil der Beleuchtungsvorrichtung vorgesehen ist, wobei das Filter eine Vorrichtung zum Befestigen des Filters an dem Handgriffelement umfasst. Die Befestigungsvorrichtung ist zum Beispiel als lösbare Klammer oder als Steckvorrichtung ausgebildet.

Vorzugsweise weist die Vorrichtung zur Erkennung einer Anomalie am Zahn und / oder zur Erkennung von gesundem Zahngewebe eine Lichtleitvorrichtung auf, die ausgebildet ist, die von dem zu untersuchenden Zahn abgestrahlte Strahlung, insbesondere die zumindest zwei voneinander getrennten Wellenlängen oder Wellenlängenbänder, von dem zu untersuchenden Zahn wegzuleiten. Bevorzugt ist die Lichtleitvorrichtung an einem Handgriffelement vorgesehen, insbesondere ist die Lichtleitvorrichtung zumindest teilweise im Inneren des Handgriffelements angeordnet. Besonders bevorzugt ist das Filter an der Lichtleitvorrichtung vorgesehen. Die Lichtleitvorrichtung leitet die von dem zu untersuchenden Zahn abgestrahlte Strahlung insbesondere in Richtung eines Detektors, einer Auswerteinheit, einer Kamera und / oder einer Anzeige, welche auf Basis der von dem zu untersuchenden Zahn abgestrahlten Strahlung ausgebildet ist, dem Anwender eine Anomalie des zu untersuchenden Zahns und / oder gesundes Zahngewebe anzuzeigen.

Vorzugsweise umfasst die Vorrichtung zur Erkennung einer Anomalie am Zahn und / oder zur Erkennung von gesundem Zahngewebe des Weiteren einen Detektor oder eine Kamera, insbesondere eine Farbkamera, die ausgebildet sind, die zumindest zwei durchgelassenen, voneinander getrennte Wellenlängen oder Wellenlängenbänder zu empfangen, zu detektieren oder zu verarbeiten. Der Detektor oder die Kamera sind bevorzugt mit einer Anzeige verbunden, insbesondere einem Bildschirm, die ausgebildet ist, auf Basis der von dem Detektor oder der Kamera erzeugten elektrischen Signale dem Anwender eine Anomalie des zu untersuchenden Zahns und / oder gesundes Zahngewebe anzuzeigen. Insbesondere bildet die Anzeige zumindest einen Teil des zu untersuchenden Zahns oder der Anomalie des Zahns oder des gesunden Zahngewebes ab.

Die von dem zu untersuchenden Zahn abgestrahlte Strahlung, insbesondere die zumindest zwei von dem Filter durchgelassenen, voneinander getrennten Wellenlängen oder die zumindest zwei voneinander getrennten Wellenlängenbänder, weist / weisen vorzugsweise Fluoreszenzstrahlung auf.

Ein Verfahren zur Erkennung einer Anomalie an einem Zahn, insbesondere zur Erkennung von Karies, Plaque oder Zahnstein, und / oder zur Erkennung von gesundem Zahngewebe oder ein Verfahren zur Aufbereitung von Strahlung, welche von einem zu untersuchenden Zahn abgestrahlten wird, ist dadurch definiert, dass ein Filter gleichzeitig zumindest zwei Wellenlängen oder Wellenlängenbänder der von dem zu untersuchenden Zahn abgestrahlten Strahlung durchlässt und zumindest jeweils eine Wellenlänge oder ein Wellenlängenband ausfiltert, welche(s) sich zwischen den durchgelassenen Wellenlängen oder Wellenlängenbändern befindet, so dass die Strahlung nach dem Durchtritt durch das Filter zumindest zwei Wellenlängen oder Wellenlängenbänder aufweist, welche durch zumindest eine ausgefilterte Wellenlänge oder zumindest ein ausgefiltertes Wellenlängenband voneinander getrennt sind.

Vorzugsweise werden bei dem Verfahren die zumindest zwei voneinander getrennten Wellenlängen oder Wellenlängenbänder gemeinsam durch das Filter und vorzugsweise gemeinsam auf einem einzigen, unmittelbar an das Filter anschließenden, optischen Pfad geleitet.

Vorzugsweise wird während des Verfahrens ein bewegliches Filter in den optischen Pfad der von dem zu untersuchenden Zahn abgestrahlte Strahlung bewegt und / oder daraus entfernt und / oder das Filter vor das Auge des Anwenders bewegt und / oder vom Auge weg bewegt.

Vorzugsweise wird das Verfahren mit einer im Vorstehenden beschriebenen Vorrichtung zur Erkennung einer Anomalie an einem Zahn, insbesondere zur Erkennung von Karies, Plaque oder Zahnstein, und / oder zur Erkennung von gesundem Zahngewebe durchgeführt, insbesondere um damit eine Anomalie an einem Zahn, insbesondere Karies, Plaque oder Zahnstein, zu erkennen. Bevorzugt umfasst das Verfahren in einem oder mehreren weiteren Schritt die Behandlung, Heilung und / oder Entfernung der Anomalie.

Die Erfindung wird nachfolgend anhand bevorzugter Ausführungsbeispiele und Bezug nehmend auf die beigefügten Zeichnungen erläutert:
Figur 1 zeigt eine schematische Darstellung eines ersten Ausführungsbeispiels einer Vorrichtung zur Erkennung einer Anomalie an einem Zahn und / oder zur Erkennung von gesundem Zahngewebe.
Figur 2 zeigt eine schematische Darstellung eines zweiten Ausführungsbeispiels einer Vorrichtung zur Erkennung einer Anomalie an einem Zahn und / oder zur Erkennung von gesundem Zahngewebe.
Figur 3 zeigt eine schematische Darstellung eines dritten Ausführungsbeispiels einer Vorrichtung zur Erkennung einer Anomalie an einem Zahn und / oder zur Erkennung von gesundem Zahngewebe, bei welcher das Filter an einem Handgriffelement befestigt ist.
Figur 4 zeigt ein erstes Ausführungsbeispiel eines Filters einer Vorrichtung zur Erkennung einer Anomalie an einem Zahn und / oder zur Erkennung von gesundem Zahngewebe, wobei das Filter eine Vorrichtung zum Befestigen des Filters an einem Anwender aufweist.
Figur 5 zeigt ein zweites Ausführungsbeispiel eines Filters einer Vorrichtung zur Erkennung einer Anomalie an einem Zahn und / oder zur Erkennung von gesundem Zahngewebe, wobei das Filter eine Vorrichtung zum Befestigen des Filters an einem Anwender und eine Haltevorrichtung aufweist.
Figur 6 zeigt ein drittes Ausführungsbeispiel eines Filters einer Vorrichtung zur Erkennung einer Anomalie an einem Zahn und / oder zur Erkennung von gesundem Zahngewebe, wobei das Filter eine Vorrichtung zum Befestigen des Filters an einem Anwender aufweist und beweglich ausgebildet ist, so dass das Filter in den optischen Pfad der von dem zu untersuchenden Zahn abgestrahlten Strahlung bewegbar und daraus entfernbar ist und / oder das Filter vor das Auge des Anwenders und vom Auge weg bewegbar ist.
Figur 7 zeigt ein Diagramm mit den Verläufen der Strahlungsintensität der von dem Zahn abgestrahlten Strahlung über die Wellenlänge bei anomalen Zahngewebe (kariösem Zahngewebe) und gesundem Zahngewebe.
Figur 8 zeigt ein Diagramm mit dem Verlauf der Transmission eines ersten Filters einer Vorrichtung zur Erkennung einer Anomalie an einem Zahn über die Wellenlänge.
Figur 9 zeigt ein Diagramm mit dem Verlauf der Transmission eines zweiten Filters einer Vorrichtung zur Erkennung einer Anomalie an einem Zahn über die Wellenlänge.
Figur 10 zeigt ein Diagramm mit dem Verlauf der Strahlungsintensität der Strahlung nach dem Verlassen des Filters über die Wellenlänge.

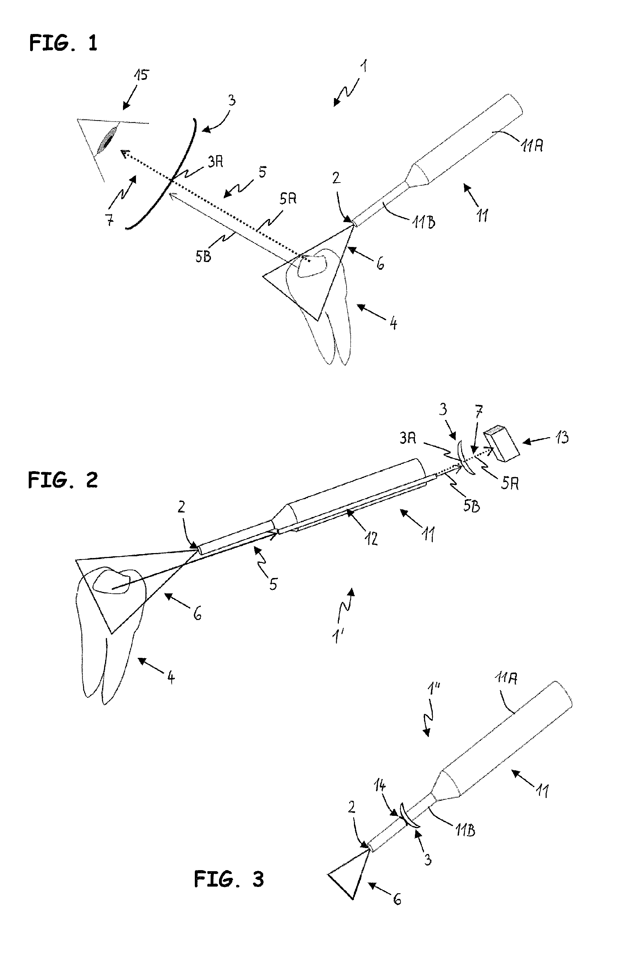

Die in der Figur 1 dargestellte Vorrichtung 1 zur Erkennung einer Anomalie an einem Zahn, insbesondere zur Erkennung von Karies, Plaque oder Zahnstein, und / oder zur Erkennung von gesundem Zahngewebe weist eine Beleuchtungsvorrichtung 2 sowie ein Filter 3 auf. Die Beleuchtungsvorrichtung 2 umfasst als Strahlungsquelle ein optisches Halbleiterelement, zum Beispiel durch eine Leuchtdiode, insbesondere einer Laserdiode. Die Beleuchtungsvorrichtung 2 emittiert elektromagnetische Strahlung zum Beispiel mit einer Wellenlänge im Bereich von etwa 380 nm - 420 nm, vorzugsweise mit etwa 405 nm.

Die Beleuchtungsvorrichtung 2 ist vorzugsweise an einem, zum Beispiel als gerades Handstück oder gebogenes Winkelstück ausgebildeten, Handgriffelement 11 vorgesehen, insbesondere im Handgriffelement 11 aufgenommen. Das Handgriffelement 11 weist einen, vorzugsweise zylindrischen, Griffabschnitt 11A, insbesondere zum Halten durch den Anwender, und einen daran anschließenden, vorzugsweise ebenfalls zylindrischen oder rohrförmigen, Vorderabschnitt 11B auf. Vorzugsweise ist der Durchmesser des Griffabschnitts 11A größer als der Durchmesser des Vorderabschnitts 11B.

Im oder am Griffabschnitt 11A sind vorzugsweise die Energiequelle (insbesondere im Falle eines kabellosen Handgriffelements 11), zum Beispiel eine Batterie oder ein mehrfach aufladbarer Akkumulator, oder Teile der Energieversorgung, zum Beispiel eine elektrische Kontakt- oder Kupplungsvorrichtung an eine externe Energiequelle, vorgesehen. Des Weiteren können in oder am Griffabschnitt 11A eine Steuervorrichtung, zum Beispiel zum Betrieb des Handgriffelements 11 oder der Beleuchtungsvorrichtung 2 mit unterschiedlichen Betriebsprogrammen, ein oder mehrere Stell-, Steuer- oder Betätigungselemente, zum Beispiel zum Ein-/Ausschalten der Beleuchtungsvorrichtung 2 oder zum Auswählen unterschiedlicher Betriebsprogramme, und / oder eine Anzeigevorrichtung zum Anzeigen von Betriebsparametern angeordnet sein.

Der Vorderabschnitt 11B, welcher insbesondere dafür vorgesehen ist, zumindest teilweise in den Mundraum eines Patienten eingeführt zu werden, umfasst vorzugsweise zumindest einen Teil der Beleuchtungsvorrichtung 2, zum Beispiel die Strahlungsquelle und / oder einen Licht- oder Strahlungsleiter, welcher von der Strahlungsquelle emittiertes Licht in Richtung des distalen, freien Endes des Vorderabschnitts 11 B oder in Richtung des zu untersuchenden Zahns 4 leitet.

Das Handgriffelement 11 oder die Beleuchtungsvorrichtung 2 emittiert Strahlung 6 (schematisch und beispielhaft durch einen Strahlungskegel symbolisiert) in Richtung und auf einen zu untersuchenden Zahn 4. Die Strahlung 6 ist derart beschaffen, dass der bestrahlte, zu untersuchende Zahn 4 oder zumindest ein Teil davon Strahlung 5 (ebenfalls schematisch und beispielhaft in der Figur 1 dargestellt) zurück- oder abstrahlen kann. Zumindest ein Teil dieser abgestrahlten Strahlung 5 oder deren Wellenlängen sind zum Nachweis einer Anomalie, insbesondere von Karies, Plaque oder Zahnstein, an dem Zahn 4 und / oder von gesundem Zahngewebe geeignet.

Aus der Figur 1 ist des Weiteren zu erkennen, dass ein einziges, insbesondere als Doppel- oder Mehrfach-Band-Pass-Filter ausgebildetes, Filter 3 vorgesehen ist, um die von dem Zahn 4 ab- oder zurückgestrahlte Strahlung 5 zu filtern. Die von dem Zahn 4 ab- oder zurückgestrahlte Strahlung 5 umfasst zwei Komponenten (welche schematisch in der Figur 1 abgebildet sind), nämlich zumindest zwei Wellenlängen oder Wellenlängen bänder 5A die von dem Filter 3 durchgelassen werden und zumindest eine Wellenlänge oder ein Wellenlängenband 5B, das vom Filter 3 nicht durchgelassen, d.h. ausgefiltert, wird. Das Filter 3 ist demgemäß ausgebildet, gleichzeitig zumindest zwei Wellenlängen oder Wellenlängenbänder 5A der von dem zu untersuchenden Zahn 4 abgestrahlten Strahlung 5 durchzulassen und zumindest jeweils eine Wellenlänge oder ein Wellenlängenband 5B zwischen den durchgelassenen Wellenlängen oder Wellenlängenbändern 5A auszufiltern (oder zu unterdrücken oder abzuschwächen), so dass die Strahlung 5 nach Verlassen des Filters 3 zumindest zwei Wellenlängen oder Wellenlängenbänder 5A aufweist, welche durch zumindest eine ausgefilterte Wellenlänge oder zumindest ein ausgefiltertes Wellenlängenband 5B voneinander getrennt sind. Das Filter 3 bildet des Weiteren vorzugsweise einen, insbesondere gemeinsamen und / oder einzigen, optischen Pfad 3A für die zumindest zwei durchgelassenen, voneinander getrennten Wellenlängen oder Wellenlängenbänder 5A.

Nach dem Durchtritt der Strahlung 5, 5A durch das Filter 3 oder nach dem Verlassen des Filters 3 wird die Strahlung 5A in oder auf einem einzigen, unmittelbar an das Filter 3 anschließenden, gemeinsamen optischen Pfad 7 für die zumindest zwei durchgelassenen, voneinander getrennten Wellenlängen oder Wellenlängenbänder 5A weitergeleitet. Über den gemeinsamen optischen Pfad 7 gelangt die Strahlung 5A gemäß der Figur 1 zu einem Auge 15 des Anwenders. Die Strahlung 5A kann nach dem Filter 3 als Freistrahl ausgebildet sein, so dass der gemeinsame optische Pfad 7 als Raumvolumen oder Umgebung zwischen dem Filter 3 und dem Auge 15 ausgebildet ist. Selbstverständlich kann der gemeinsame optische Pfad 7 jedoch auch einen beliebigen Strahlungs- oder Lichtleiter oder andere optische Elemente umfassen.

Die Vorrichtungen 1', 1" zur Erkennung einer Anomalie an einem Zahn, insbesondere zur Erkennung von Karies, Plaque oder Zahnstein, und / oder zur Erkennung von gesundem Zahngewebe der Figuren 2 und 3 gleichen in vielen Merkmalen der im Vorstehenden beschriebenen Vorrichtung 1, so dass im Folgenden vor allem die Unterschiede zwischen den Vorrichtungen 1, 1', 1" erläutert werden. Gleiche Bauteile der Vorrichtungen 1, 1', 1" oder gleiche Merkmale in den Figuren 1 - 3 tragen dieselben Bezugszeichen.

An oder in dem Handgriffelement 11 der Figur 2 ist eine Lichtleitvorrichtung 12 vorgesehen, die ausgebildet ist, zumindest einen Teil der von dem zu untersuchenden Zahn 4 abgestrahlten Strahlung 5, insbesondere die zumindest zwei voneinander getrennten Wellenlängen oder Wellenlängenbänder 5A, von dem zu untersuchenden Zahn 4 wegzuleiten. Die Lichtleitvorrichtung 12 weist zumindest einen Lichtleiter auf, zum Beispiel einen Glasstab oder einen optischen Faserstab. Die Vorrichtung 1' umfasst des Weiteren ein Filter 3, das wie im Vorstehenden bereits im Detail beschrieben, ausgebildet ist, gleichzeitig zumindest zwei Wellenlängen oder Wellenlängenbänder 5A der von dem zu untersuchenden Zahn 4 abgestrahlten Strahlung 5 durchzulassen und zumindest jeweils eine Wellenlänge oder ein Wellenlängenband 5B zwischen den durchgelassenen Wellenlängen oder Wellenlängenbändern 5A auszufiltern, so dass die Strahlung 5 nach Verlassen des Filters 3 zumindest zwei Wellenlängen oder Wellenlängenbänder 5A aufweist, welche durch zumindest eine ausgefilterte Wellenlänge oder zumindest ein ausgefiltertes Wellenlängenband 5B voneinander getrennt sind. Das Filter ist zum Beispiel am Handgriffelement 11 befestigt und / oder mit der Lichtleitvorrichtung 12 verbunden und / oder als Teil der Lichtleitvorrichtung 12 ausgebildet.

Nach dem Durchtritt durch das Filter 3 gelangt die Strahlung 5A, d.h. die zumindest zwei voneinander getrennten Wellenlängen oder Wellenlängenbänder, via einem gemeinsamen optischen Pfad 7 zu einem Detektor oder einer Kamera 13, insbesondere einer Farbkamera, die ausgebildet sind, die zumindest zwei durchgelassenen, voneinander getrennte Wellenlängen oder Wellenlängenbänder 5A zu empfangen, zu detektieren oder zu verarbeiten. Vorzugsweise werden die von dem Detektor oder einer Kamera 13 erzeugten elektrischen Signale an eine Anzeige übertragen, insbesondere an einen Bildschirm, die ausgebildet ist, auf Basis der von dem Detektor oder der Kamera erzeugten elektrischen Signale dem Anwender eine Anomalie des zu untersuchenden Zahns und / oder gesundes Zahngewebe anzuzeigen.

Bei der Vorrichtung 1" der Figur 3 ist das Filter 3, vorzugsweise lösbar, am Handgriffelement 11 befestigt. Dazu ist am Handgriffelement 11 und / oder am Filter 3, eine vorzugsweise lösbare, Befestigungsvorrichtung 14 vorgesehen, zum Beispiel eine Steckvorrichtung, insbesondere eine das Handgriffelement 3 zumindest teilweise umgreifende Klammer, oder eine Klemmvorrichtung zum Klemmen des Filters 3 am Handgriffelement 11. Gemäß der Figur 3 ist das Filter 3 am Vorderabschnitt 11 B befestigt, es kann jedoch klarerweise an jedem Teil des Handgriffelements 11 vorgesehen sein, insbesondere auch am Griffabschnitt 11A. Das Filter 3 ist, wie im Vorstehenden bereits im Detail beschrieben, wiederum ausgebildet, gleichzeitig zumindest zwei Wellenlängen oder Wellenlängenbänder 5A der von dem zu untersuchenden Zahn 4 abgestrahlten Strahlung 5 durchzulassen und zumindest jeweils eine Wellenlänge oder ein Wellenlängenband 5B zwischen den durchgelassenen Wellenlängen oder Wellenlängenbändern 5A auszufiltern, so dass die Strahlung 5 nach Verlassen des Filters 3 zumindest zwei Wellenlängen oder Wellenlängenbänder 5A aufweist, welche durch zumindest eine ausgefilterte Wellenlänge oder zumindest ein ausgefiltertes Wellenlängenband 5B voneinander getrennt sind.

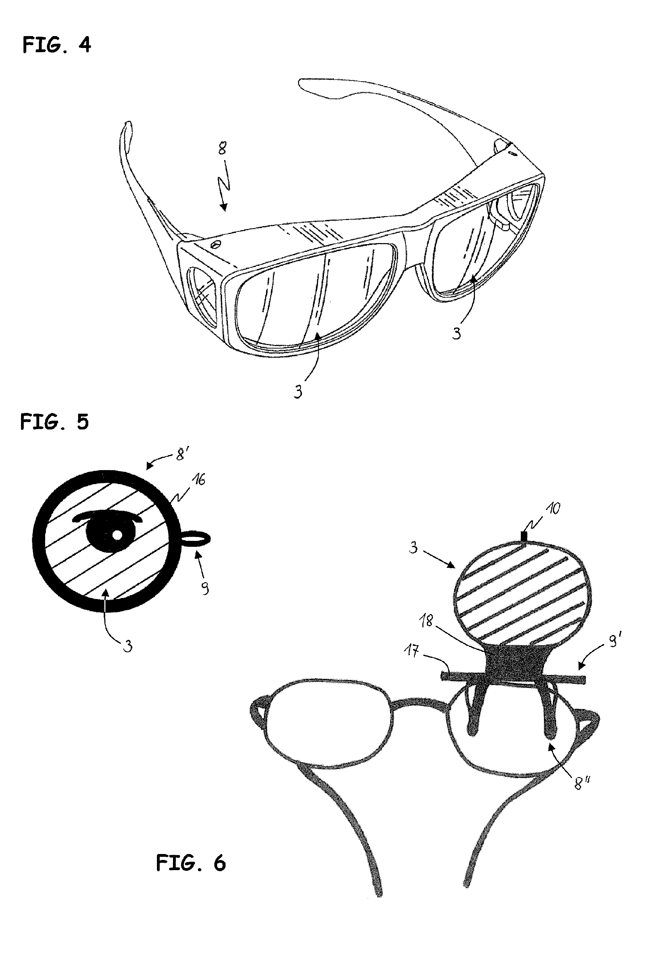

Die Figuren 4 - 6 zeigen unterschiedliche Ausführungsbeispiele des Filters 3 oder der Art, wie das Filter 3 mit einer Vorrichtung zum Befestigen des Filters 3 an einem Anwender der Vorrichtung 1, 1', 1" oder mit einer Vorrichtung zum Halten durch den Anwender vorsehen sein kann. Die Befestigungs- oder Haltevorrichtungen sind insbesondere mit der in der Figur 1 dargestellten Vorrichtung 1 verwendbar oder kombinierbar.

Die Figur 4 zeigt ein erstes Ausführungsbeispiel einer Vorrichtung 8 zum Befestigen des Filters 3 an einem Anwender der Vorrichtung 1. Die Befestigungsvorrichtung 8 ist am Kopf des Anwenders befestigbar und weist vorzugsweise zwei, insbesondere idente, Filter 3 auf. Die Befestigungsvorrichtung 8 ist insbesondere als Brillengestell ausgebildet.

Die Figur 5 zeigt ein Filter 3, das an seinem Umfang oder seiner Außenkante von einer Fassung oder einem Rahmen 16 umgeben ist. Der Rahmen 16 bildet zumindest einen Teil einer Vorrichtung 8' zum Befestigen des Filters 3 an einem Anwender der Vorrichtung 1. Das Filter 3 kann vorzugsweise im Sinne eines Monokels verwendet werden, wobei das Filter 3 mittels der Befestigungsvorrichtung 8' oder der Fassung 16 am Auge, insbesondere mittels des Augenlidmuskels, geklemmt wird. Des Weiteren ist das Filter 3 oder die Befestigungsvorrichtung 8' oder der Rahmen 16 mit einer Vorrichtung 9 zum Halten durch den Anwender vorsehen. Mittels der Haltevorrichtung 9 kann der Anwender das Filter 3, zum Beispiel mit der Hand, halten, ohne das Filter 3 selbst zu berühren. Die Haltevorrichtung 9 ist zum Beispiel als Griffteil oder als Öse ausgebildet.

Die Figur 6 zeigt ein beweglich ausgebildetes Filter 3, insbesondere beweglich relativ zu der Befestigungsvorrichtung 8" zum Befestigen des Filters 3 an dem Anwender oder zu der Haltevorrichtung 9' oder zu dem gemeinsamen optischen Pfad 7 der Strahlung 5A nach dem Filter 3. Dazu ist das Filter 3 vorzugsweise mit einer Drehachse 17 verbunden, zum Beispiel in Form eines Stabs, um die das Filter 3 drehbar ist. Das Filter 3 ist zum Beispiel über ein Verbindungselement 18 mit der Drehachse / dem Stab 17 verbunden, wobei das Verbindungselement 18 eine Bohrung aufweist, in welcher der Stab 17 aufgenommen und gelagert ist, so dass das Filter 3 mit dem Verbindungselement 18 um die Drehachse / den Stab 17 drehbar ist. Aufgrund der Beweglichkeit des Filters 3 ist dieser nun wahlweise vor die Brille, an welcher das Filter 3 gemäß der Figur 6 befestigt ist, und / oder das Auge des Anwenders und / oder in den optischen Pfad 7 der von dem zu untersuchenden Zahn 4 abgestrahlten Strahlung 5 bewegbar und davon / daraus entfernbar.

Das Filter 3 ist wiederum mit einer Befestigungsvorrichtung 8" verbunden, so dass das Filter 3 direkt oder indirekt am Anwender befestigbar ist - gemäß der Figur 6 indirekt über eine Brille. Das Filter 3 ist somit auch relativ zur Befestigungsvorrichtung 8" bewegbar. Vorzugsweise dient der Stab 17 auch als Haltevorrichtung 9', so dass der Anwender das Filter 3 indirekt mit der Hand halten oder ergreifen kann. Selbstverständlich kann die Haltevorrichtung 9' jedoch auch als vollständig separate Vorrichtung ausgebildet sein, so dass der Stab 17 nur als Drehachse des Filters 3 dient. Selbstverständlich kann auch die Bewegungsrichtung des Filters 3 anders als in der Figur 6 ausgebildet sein, zum Beispiel kann der Stab 17 vertikal angeordnet sein, so dass das Filter 3 seitwärts, in Richtung des Bügels der Brille bewegbar ist.

Um das Filter 3 nicht berühren zu müssen und dadurch zu verschmutzen, ist das Filter 3 vorzugsweise mit einem Betätigungselement 10 versehen, zum Beispiel in Form eines Fortsatzes oder eines kurzen Stabs, mit Hilfe dessen der Anwender das Filter 3 relativ zum Auge oder zur Befestigungsvorrichtung 8" oder zu dem optischen Pfad 7 bewegen kann. Bevorzugt ist das Betätigungselement 10 lösbar mit dem Filter 3 verbunden und insbesondere als Einwegvorrichtung ausgebildet.

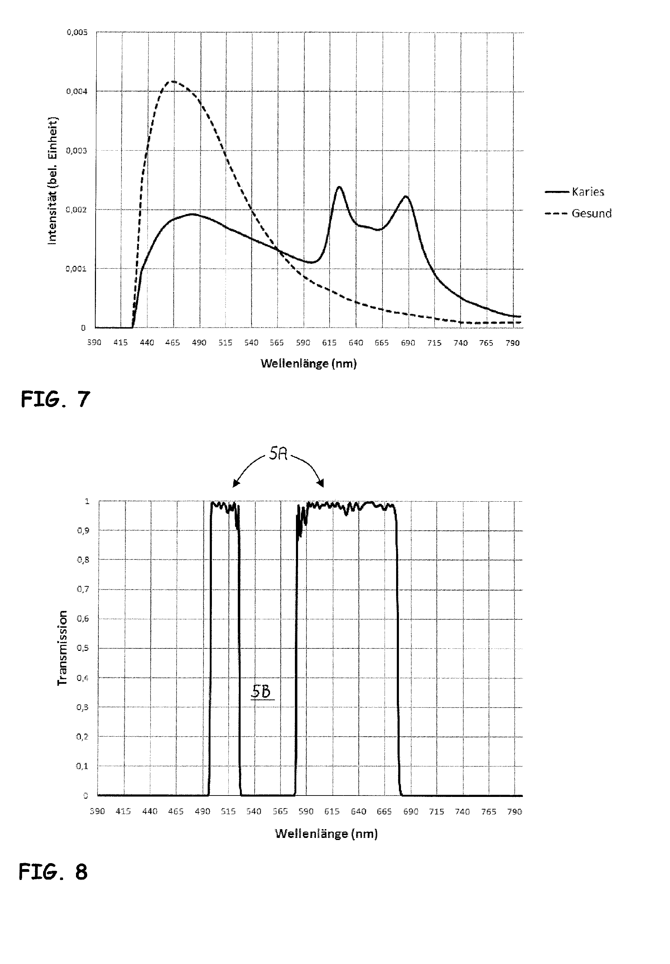

Das Diagramm der Figur 7 zeigt die Verläufe der Strahlungsintensität der von dem Zahn 4 abgestrahlten Strahlung 5 über die Wellenlänge bei anomalen Zahngewebe (insbesondere kariösem Zahngewebe) und gesundem Zahngewebe. Es ist zu erkennen, dass die von gesundem Zahngewebe abgestrahlte Strahlung eine Intensitätsspitze im Bereich von etwa 430 nm bis etwa 540 nm und die von anomalem, kariösem Zahngewebe abgestrahlte Strahlung eine oder mehrere Intensitätsspitzen im Bereich von etwa 615 nm bis etwa 700 nm aufweist, wobei die Intensität der Strahlung des gesunden Zahngewebes im Spitzenbereich von etwa 430 nm bis etwa 540 nm deutlich höher ist als die Intensität der Strahlung des anomalen, kariösen Zahngewebes in dessen Spitzenbereich von etwa 615 nm bis etwa 700 nm. Aufgrund der hohen Intensität der Strahlung des gesunden Zahngewebes ist eine zuverlässige Erkennung von anomalem, kariösem Zahngewebe nur unter Verwendung eines geeigneten Filters möglich.

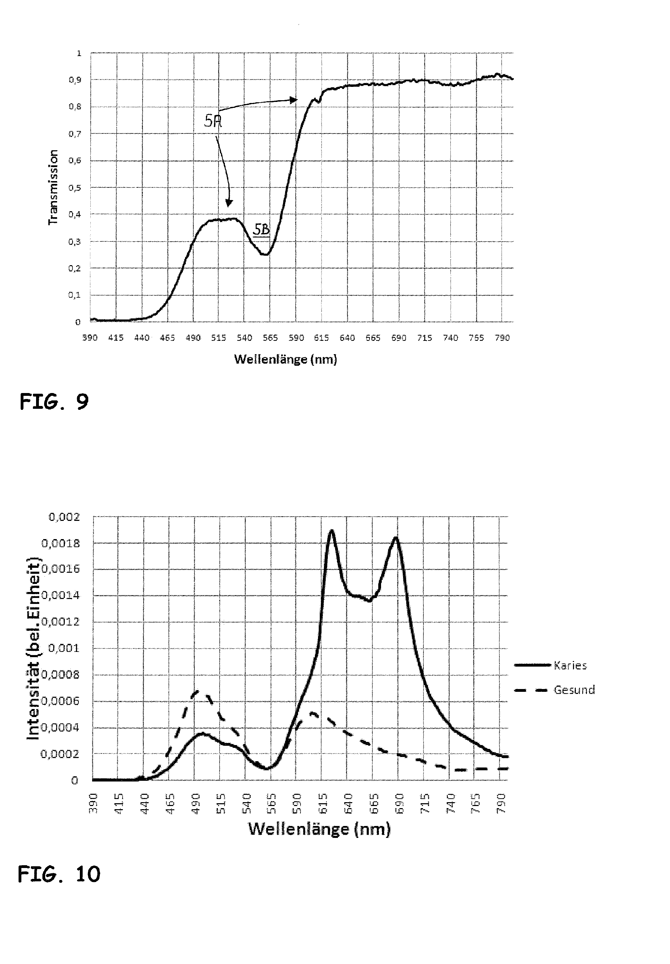

Die Figuren 8 und 9 zeigen Diagramme mit dem Verlauf der Transmission eines ersten und eines zweiten Filters 3 einer Vorrichtung 1, 1', 1" zur Erkennung einer Anomalie an einem Zahn über die Wellenlänge. Das Filter 3 der Figur 8, zum Beispiel in Form eines Interferenzfilters, ist ausgebildet, ein erstes Wellenlängenband im Bereich von etwa 490 nm bis etwa 530 nm und ein zweites Wellenlängenband im Bereich von etwa 575 nm bis etwa 680 nm durchzulassen und alle weiteren Wellenlängen im Wesentlichen auszufiltern oder alle weiteren Wellenlängen, insbesondere deren Intensität, zumindest in Relation zu den beiden durchgelassenen Wellenlängenbändern erheblich zu reduzieren. Nach dem Filter 3 oder nach Verlassen des Filters 3 sind die beiden durchgelassenen Wellenlängenbänder 5A somit durch ein ausgefiltertes Wellenlängenband 5B im Bereich von etwa 530 nm bis etwa 575 nm getrennt. Das zweite Wellenlängenband im Bereich von etwa 575 nm bis etwa 680 nm umfasst dabei im Wesentlichen den Intensitäts-Spitzenbereich von etwa 615 nm bis etwa 700 nm der Strahlung des anomalen, kariösen Zahngewebes (siehe Figur 7), so dass das anomale, kariösen Zahngewebe für den Anwender deutlich erkennbar ist. Das erste Wellenlängenband im Bereich von etwa 490 nm bis etwa 530 nm liegt im Bereich des Intensitäts-Spitzenbereichs des gesunden Zahngewebes und damit im Bereich der höchsten Intensität und verbessert damit erheblich die Erkennbarkeit der Anomalität des Zahns, insbesondere den visuellen Kontrast für den Anwender.

Das Diagramm der Figur 9 zeigt ein Ausführungsbeispiel eines Verlaufs der Transmission eines zweiten Filters 3, zum Beispiel eines Kunststofffilters. Es ist zu erkennen, dass die prinzipielle Transmissionseigenschaft (d.h. das Durchlassen zweier Wellenlängenbänder 5A und das Ausfiltern eines zwischen diesen beiden durchgelassenen Wellenlängenbänder 5A liegenden Wellenlängenbands 5B) des Filters der Figur 9 jener des Filters der Figur 8 gleicht, jedoch sind auch mehrere Unterschiede bemerkbar: Insbesondere ist die Transmission des Filters der Figur 9 für das zwischen den beiden durchgelassenen Wellenlängenbänder 5A liegende Wellenlängenband 5B höher als die Transmission des Filters der Figur 8, so dass zumindest ein geringer Anteil der Wellenlängen des Wellenlängenbands 5B durch das Filter hindurchtritt. Des Weiteren ist die Transmission für die Wellenlängen der beiden durchgelassenen Wellenlängenbänder 5A deutlich unterschiedlich, wobei insbesondere die Transmission der Strahlung des anomalen, kariösen Zahngewebes in dessen Spitzenbereich von etwa 615 nm bis etwa 700 nm merklich höher ist als die Transmission der Strahlung des gesunden Zahngewebes im Bereich von etwa 430 nm bis etwa 540 nm.

Selbstverständlich sind die im Vorstehenden angeführten Werte der Wellenlängen, Wellenlängenbereiche und / oder Wellenlängenbänder nur Beispielswerte, welche zur verbesserten Verständlichkeit angeführt sind. Diese Werte sind variabel, so wie dies im Vorstehenden auch schon angeführt wurde. Wesentlich ist, dass das Filter 3 ausgebildet ist gleichzeitig zumindest zwei Wellenlängen oder Wellenlängenbänder 5A der von dem zu untersuchenden Zahn 4 abgestrahlten Strahlung 5 durchzulassen und zumindest jeweils eine Wellenlänge oder ein Wellenlängenband 5B zwischen den durchgelassenen Wellenlängen oder Wellenlängenbändern 5A (vollständig oder teilweise, insbesondere signifikant) auszufiltern (oder zu unterdrücken oder abzuschwächen), so dass die Strahlung 5 nach Verlassen des Filters 3 zumindest zwei Wellenlängen oder Wellenlängenbänder 5A aufweist, welche durch zumindest eine ausgefilterte Wellenlänge oder zumindest ein ausgefiltertes Wellenlängenband 5B voneinander getrennt sind. Wie aus dem Vorstehenden, insbesondere in Verbindung mit den Figuren 7 - 9, zu erkennen ist, umfassen die beiden durchgelassenen Wellenlängen oder Wellenlängenbänder 5A dabei vorzugsweise zumindest Abschnitte oder Teile der Intensitätsspitzen der Strahlung des gesunden Zahngewebes und der Strahlung des anomalen, insbesondere kariösen, Zahngewebes.

Das Diagramm der Figur 10 zeigt schließlich den Verlauf der Strahlungsintensität der Strahlung 5 nach dem Verlassen des Filters 3 über die Wellenlänge, insbesondere für ein Filter mit ähnlichen Transmissionseigenschaften wie das Filter der Figur 9. Im Vergleich zum Verlauf der Strahlungsintensität der Figur 7 übertrifft aufgrund der Wirkung des Filters 3 der Wert der Intensität der Strahlung des anomalen, kariösen Zahngewebes den Wert der Intensität der Strahlung des gesunden Zahngewebes erheblich. Unter Berücksichtigung der Empfindlichkeit des menschlichen Auges, insbesondere der höheren Empfindlichkeit im Bereich der Strahlung des gesunden Zahngewebes, zum Beispiel im Bereich von etwa 430 nm bis etwa 540 nm, ist damit anomales Zahngewebe für den Anwender einfach und zuverlässig zu erkennen.

Die Erfindung ist nicht auf die beschriebenen Ausführungsbeispiele beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Vorrichtung (1, 1', 1") zur Erkennung einer Anomalie an einem Zahn (4), insbesondere zur Erkennung von Karies, Plaque oder Zahnstein, und / oder zur Erkennung von gesundem Zahngewebe, umfassend: Eine Beleuchtungsvorrichtung (2), die zur Abgabe von Strahlung (6) auf zumindest einen zu untersuchenden Zahn (4) ausgebildet ist, derart, dass der bestrahlte, zu untersuchende Zahn (4) Strahlung (5) abstrahlt, und ein Filter (3), das ausgebildet ist, von dem zu untersuchenden Zahn (4) abgestrahlte Strahlung (5) durchzulassen, deren Wellenlänge zum Nachweis einer Anomalie an einem Zahn (4) geeignet ist, **dadurch gekennzeichnet, dass**
das Filter (3) ausgebildet ist, gleichzeitig zumindest zwei Wellenlängen oder Wellenlängenbänder (5A) der von dem zu untersuchenden Zahn (4) abgestrahlten Strahlung (5) durchzulassen und zumindest jeweils eine Wellenlänge oder ein Wellenlängenband (5B) zwischen den durchgelassenen Wellenlängen oder Wellenlängenbändern (5A) auszufiltern, so dass die Strahlung (5) nach Verlassen des Filters (3) zumindest zwei Wellenlängen oder Wellenlängenbänder (5A) aufweist, welche durch zumindest eine ausgefilterte Wellenlänge oder zumindest ein ausgefiltertes Wellenlängenband (5B) voneinander getrennt sind.

2. Vorrichtung (1, 1', 1") nach Anspruch 1, **dadurch gekennzeichnet, dass**
das Filter (3) einen optischen Pfad (3A) für die zumindest zwei durchgelassenen, voneinander getrennten Wellenlängen oder Wellenlängenbänder (5A) bildet.

3. Vorrichtung (1, 1', 1") nach Anspruch 1 oder 2, **gekennzeichnet durch**
einen einzigen, unmittelbar an das Filter (3) anschließenden, gemeinsamen optischen Pfad (7) für die zumindest zwei durchgelassenen, voneinander getrennten Wellenlängen oder Wellenlängenbänder (5A).

4. Vorrichtung (1, 1', 1") nach einem der vorstehenden Ansprüche, **dadurch gekennzeichnet, dass**
das Filter (3) als Mehrfach-Band-Pass-Filter, insbesondere als Doppel-Band-Pass-Filter, ausgebildet ist.

5. Vorrichtung (1, 1', 1") nach einem der vorstehenden Ansprüche, **dadurch gekennzeichnet, dass**
an dem Filter (3) eine Vorrichtung (8, 8', 8") zum Befestigen des Filters (3) an einem Anwender der Vorrichtung (1, 1', 1") zur Erkennung einer Anomalie an einem Zahn (4) und / oder eine Vorrichtung (9, 9') zum Halten durch den Anwender vorgesehen sind, zum Beispiel ein Brillengestell oder ein Griffteil.

6. Vorrichtung (1, 1', 1") nach einem der vorstehenden Ansprüche, **dadurch gekennzeichnet, dass**
das Filter (3) beweglich ausgebildet ist, insbesondere beweglich relativ zu der Vorrichtung (8, 8', 8") zum Befestigen des Filters (3) an dem Anwender oder zu der Vorrichtung (9, 9') zum Halten durch den Anwender, so dass das Filter (3) in den optischen Pfad der von dem zu untersuchenden Zahn (4) abgestrahlten Strahlung (5) bewegbar und daraus entfernbar ist und / oder das Filter (3) vor das Auge des Anwenders und vom Auge weg bewegbar ist.

7. Vorrichtung (1, 1', 1") nach einem der vorstehenden Ansprüche, **dadurch gekennzeichnet, dass**
das Filter (3) und / oder die Vorrichtung (8, 8', 8") zum Befestigen des Filters (3) an dem Anwender und / oder die Vorrichtung (9, 9') zum Halten durch den Anwender und / oder ein Betätigungselement (10) zum Bewegen des Filters (3) als Einwegvorrichtung(en) ausgebildet ist / sind.

8. Vorrichtung (1, 1', 1") nach einem der vorstehenden Ansprüche, **dadurch gekennzeichnet, dass**
die Vorrichtung (1, 1', 1") ein Handgriffelement (11) aufweist, in welchem zumindest ein Teil der Beleuchtungsvorrichtung (2) vorgesehen ist, wobei das Filter (3) eine Vorrichtung (14) zum Befestigen des Filters (3) an dem Handgriffelement (11) umfasst.

9. Vorrichtung (1, 1', 1") nach einem der vorstehenden Ansprüche, **gekennzeichnet durch**
eine Lichtleitvorrichtung (12), die ausgebildet ist, die von dem zu untersuchenden Zahn (4) abgestrahlte Strahlung (5), insbesondere die zumindest zwei voneinander getrennten Wellenlängen oder Wellenlängenbänder (5A), von dem zu untersuchenden Zahn (4) wegzuleiten.

10. Vorrichtung (1, 1', 1") nach einem der vorstehenden Ansprüche, **gekennzeichnet durch**
einen Detektor oder eine Kamera (13), insbesondere eine Farbkamera, die ausgebildet sind, die zumindest zwei durchgelassenen, voneinander getrennte Wellenlängen oder Wellenlängenbänder (5A) zu empfangen, zu detektieren oder zu verarbeiten.

11. Vorrichtung (1, 1', 1") nach einem der vorstehenden Ansprüche, **dadurch gekennzeichnet, dass**
das Filter (3) zumindest ein, vorzugsweise folienartiges, Trägermaterial aufweist, zum Beispiel Glas, Kunststoff oder Gelatine, auf welchem zumindest eine dielektrische Schicht aufgetragen ist oder welches mit Farbpigmenten versehen ist.

12. Vorrichtung (1, 1', 1") nach einem der vorstehenden Ansprüche, **dadurch gekennzeichnet, dass**
die zumindest zwei von dem Filter (3) durchgelassenen Wellenlängenbänder in etwa folgende Bereiche umfassen: 480 nm - 490 nm; 480 nm - 550 nm; 510 nm - 530 nm; 540 nm - 550 nm; 600 nm - 670 nm.

13. Vorrichtung (1, 1', 1") nach einem der vorstehenden Ansprüche, **dadurch gekennzeichnet, dass**
die von dem zu untersuchenden Zahn (4) abgestrahlte Strahlung (5), insbesondere die zumindest zwei von dem Filter (3) durchgelassenen, voneinander getrennten Wellenlängen oder die zumindest zwei voneinander getrennten Wellenlängenbänder (5A), Fluoreszenzstrahlung aufweist / aufweisen.

14. Verfahren zur Aufbereitung von Strahlung (5), welche von einem zu untersuchenden Zahn (4) abgestrahlten wird, **dadurch gekennzeichnet, dass**
ein Filter (3) gleichzeitig zumindest zwei Wellenlängen oder Wellenlängenbänder (5A) der von dem zu untersuchenden Zahn (4) abgestrahlten Strahlung (5) durchlässt und zumindest jeweils eine Wellenlänge oder ein Wellenlängenband (5B) ausfiltert, welche(s) sich zwischen den durchgelassenen Wellenlängen oder Wellenlängenbändern (5A) befindet, so dass die Strahlung (5) nach dem Durchtritt durch das Filter (3) zumindest zwei Wellenlängen oder Wellenlängenbänder (5A) aufweist, welche durch zumindest eine ausgefilterte Wellenlänge oder zumindest ein ausgefiltertes Wellenlängenband (5B) voneinander getrennt sind.

15. Verfahren zur Aufbereitung von Strahlung (5) nach Anspruch 14, **dadurch gekennzeichnet, dass**
die zumindest zwei voneinander getrennten Wellenlängen oder Wellenlängenbänder (5A) gemeinsam durch das Filter (3) und vorzugsweise gemeinsam auf einem einzigen, unmittelbar an das Filter (3) anschließenden, optischen Pfad (7) geleitet werden.

## Geänderte Patentansprüche

### Geänderte Patentansprüche gemäss Regel 137(2) EPÜ.

**1.** Vorrichtung (1, 1', 1") zur Erkennung einer Anomalie an einem Zahn (4), insbesondere zur Erkennung von Karies, Plaque oder Zahnstein, und / oder zur Erkennung von gesundem Zahngewebe, umfassend: Eine Beleuchtungsvorrichtung (2), die zur Abgabe von Strahlung (6) auf zumindest einen zu untersuchenden Zahn (4) ausgebildet ist, derart, dass der bestrahlte, zu untersuchende Zahn (4) Strahlung (5) abstrahlt, und ein Filter (3), das ausgebildet ist, von dem zu untersuchenden Zahn (4) abgestrahlte Strahlung (5) durchzulassen, deren Wellenlänge zum Nachweis einer Anomalie an einem Zahn (4) geeignet ist, wobei das Filter (3) ausgebildet ist, gleichzeitig zumindest zwei Wellenlängen oder Wellenlängenbänder (5A) der von dem zu untersuchenden Zahn (4) abgestrahlten Strahlung (5) durchzulassen und zumindest jeweils eine Wellenlänge oder ein Wellenlängenband (5B) zwischen den durchgelassenen Wellenlängen oder Wellenlängenbändem (5A) auszufiltern, so dass die Strahlung (5) nach Verlassen des Filters (3) zumindest zwei Wellenlängen oder Wellenlängenbänder (5A) aufweist, welche durch zumindest eine ausgefilterte Wellenlänge oder zumindest ein ausgefiltertes Wellenlängenband (5B) voneinander getrennt sind, wobei an dem Filter (3) eine Vorrichtung (8, 8', 8") zum Befestigen des Filters (3) an einem Anwender der Vorrichtung (1, 1', 1") zur Erkennung einer Anomalie an einem Zahn (4) und / oder eine Vorrichtung (9, 9') zum Halten durch den Anwender vorgesehen sind, **dadurch gekennzeichnet, dass**
das Filter (3) relativ zu der Vorrichtung (8, 8', 8") zum Befestigen des Filters (3) an dem Anwender oder relativ zu der Vorrichtung (9, 9') zum Halten durch den Anwender beweglich ausgebildet ist, so dass das Filter (3) in den optischen Pfad der von dem zu untersuchenden Zahn (4) abgestrahlten Strahlung (5) bewegbar und daraus entfernbar ist und / oder das Filter (3) vor das Auge des Anwenders und vom Auge weg bewegbar ist.

**2.** Vorrichtung (1, 1', 1") nach Anspruch 1, **dadurch gekennzeichnet, dass** das Filter (3) einen optischen Pfad (3A) für die zumindest zwei durchgelassenen, voneinander getrennten Wellenlängen oder Wellenlängenbänder (5A) bildet.

**3.** Vorrichtung (1, 1', 1") nach Anspruch 1 oder 2, **gekennzeichnet durch** einen einzigen, unmittelbar an das Filter (3) anschließenden, gemeinsamen optischen Pfad (7) für die zumindest zwei durchgelassenen, voneinander getrennten Wellenlängen oder Wellenlängenbänder (5A).

**4.** Vorrichtung (1, 1', 1") nach einem der vorstehenden Ansprüche, **dadurch gekennzeichnet, dass**
das Filter (3) als Mehrfach-Band-Pass-Filter, insbesondere als Doppel-Band-Pass-Filter, ausgebildet ist.

**5.** Vorrichtung (1, 1', 1") nach Anspruch 4, **dadurch gekennzeichnet, dass** das Filter (3) ausgebildet ist, ein grünes und ein davon getrenntes rotes Wellenlängenband oder ein blau-grünes und ein davon getrenntes rotes Wellenlängenband durchzulassen.

**6.** Vorrichtung (1, 1', 1") nach einem der vorstehenden Ansprüche, **dadurch gekennzeichnet, dass**
die Vorrichtung (8, 8', 8") zum Befestigen des Filters (3) an dem Anwender ein Brillengestell oder die Vorrichtung (9, 9') zum Halten durch den Anwender ein Griffteil umfasst.

**7.** Vorrichtung (1, 1', 1") nach einem der vorstehenden Ansprüche, **dadurch gekennzeichnet, dass**
das Filter (3) und / oder die Vorrichtung (8, 8', 8") zum Befestigen des Filters (3) an dem Anwender und / oder die Vorrichtung (9, 9') zum Halten durch den Anwender und / oder ein Betätigungselement (10) zum Bewegen des Filters (3) als Einwegvorrichtung(en) ausgebildet ist / sind.

**8.** Vorrichtung (1, 1', 1") nach einem der vorstehenden Ansprüche, **dadurch gekennzeichnet, dass**
die Vorrichtung (1, 1', 1") ein Handgriffelement (11) aufweist, in welchem zumindest ein Teil der Beleuchtungsvorrichtung (2) vorgesehen ist.

**9.** Vorrichtung (1, 1', 1") nach Anspruch 5,**dadurch gekennzeichnet, dass** das Filter (3) klappbar auf dem Brillengestell befestigt ist.

**10.** Vorrichtung (1, 1', 1") nach einem der vorstehenden Ansprüche, **dadurch gekennzeichnet, dass**
das Filter (3) mit einem Betätigungselement (10) versehen ist, zum Beispiel in Form eines Fortsatzes oder eines kurzen Stabs, mit Hilfe dessen der Anwender das Filter (3) relativ zum Auge oder zur Befestigungsvorrichtung (8") oder zu dem optischen Pfad (7) bewegen kann.

**11.** Vorrichtung (1, 1', 1") nach einem der vorstehenden Ansprüche, **dadurch gekennzeichnet, dass**
das Filter (3) zumindest ein, vorzugsweise folienartiges, Trägermaterial aufweist, zum Beispiel Glas, Kunststoff oder Gelatine, auf welchem zumindest eine dielektrische Schicht aufgetragen ist oder welches mit Farbpigmenten versehen ist.

**12.** Vorrichtung (1, 1', 1") nach einem der vorstehenden Ansprüche, **dadurch gekennzeichnet, dass**
die zumindest zwei von dem Filter (3) durchgelassenen Wellenlängenbänder in etwa folgende Bereiche umfassen: 480 nm - 490 nm; 480 nm - 550 nm; 510 nm - 530 nm; 540 nm - 550 nm; 600 nm - 670 nm.

**13.** Vorrichtung (1, 1', 1") nach einem der vorstehenden Ansprüche, **dadurch gekennzeichnet, dass**
die von dem zu untersuchenden Zahn (4) abgestrahlte Strahlung (5), insbesondere die zumindest zwei von dem Filter (3) durchgelassenen, voneinander getrennten Wellenlängen oder die zumindest zwei voneinander getrennten Wellenlängenbänder (5A), Fluoreszenzstrahlung aufweist / aufweisen.
